# EUROPEAN PATENT SPECIFICATION

(11) **EP 2 387 760 B1**
(45) Date of publication and mention of the grant of the patent: **01.05.2019**
(21) Application number: 10700786.6
(22) Date of filing: 14.01.2010
(51) Int. Cl.: G16H 50/50

(54) **INTERACTIVE SIMULATION OF BIOLOGICAL TISSUE**
INTERAKTIVE SIMULATION VON BIOLOGISCHEM GEWEBE
SIMULATION INTERACTIVE DE TISSU BIOLOGIQUE

(30) Priority: 15.01.2009 US 144893 P
(43) Date of publication of application: 23.11.2011
(73) Proprietor: SimQuest LLC, Maryland 21403 (US)
(72) Inventor: MEGLAN, Dwight, Westwood, Massachusetts 02090 (US); DVORNIK, Albert, Sommerville, Massachusetts 02144 (US); LENOIR, Julien, F-62130 Roellecourt (FR); SHERMAN, Paul, Seattle, Washington 98103 (US)
(74) Representative: Beck Greener
(86) International application number: PCT/US2010/020985
(87) International publication number: WO 2010/083272

(56) References cited:
- WO-A2-2007/019546
- FR-A1- 2 855 293
- US-A1- 2008 154 246

## Description

### BACKGROUND OF THE INVENTION

### 1. Field of the Invention

The present invention relates generally to the simulation of physical interactions with biological tissue.

### 2. Description of Related Art

Despite the focus on creating surgical simulators for minimally-invasive surgery (MIS) procedures, most surgery is, and will continue to be, done through open-access incisions. The work done for MIS simulators does not readily transfer to open surgery. An open simulator is more complex than one for MIS because the surgeon interacts with the body in larger surgical fields via handheld tools while directly observing his/her hand actions. This makes for potentially more complex tool-tissue interactions that encompass more anatomy than in MIS as well as for a different form of human user interface device configuration (e.g., stereo display and haptic devices) to enable direct visualization of the hands.

The majority of open surgery activities involve a tool held in the physician's hand. The tool does the actual interaction with tissue. Thus, the force and moment felt by the physician through the handle of the tool are preferably reproduced to provide a more realistic experience. This force and moment combination is felt primarily through the stressing of the joints and pressure applied by the tool handles on the skin of the tool user. This is commonly referred to as haptic sensing.

One common interaction between the tool jaws and the simulated tissue is grasping of soft-tissue, such as skin. Prior art simulators are ill-suited to accurately or quickly simulate such grasping.

### SUMMARY OF EMBODIMENTS OF THE INVENTION

According to a first aspect of the present invention there is provided a method for simulating interaction between a surgical tool and biological tissue, the method comprising: receiving tool input device information representative of movement of a physical representation of a portion of a simulated surgical tool; simulating, based on the tool input device information, an interaction between the simulated surgical tool and a simulated biological tissue, the simulating comprising using a plurality of simultaneously running computational threads for calculating the mechanics of the simulated biological tissue, at least some of the threads providing their calculations to one or more associated threads for use in such associated threads' calculations, wherein the plurality of threads comprise at least two threads that have different spatial resolutions, including a first spatial resolution and a second spatial resolution lower than the first resolution, wherein the first higher spatial resolution is utilised in respect of an area where a user is interacting; and displaying on a display a visual representation of the simulated interaction between the simulated surgical tool and the simulated biological tissue, the visual representation being based on the calculations of the plurality of threads.

According to a second aspect of the present invention there is provided a machine-readable medium encoded with machine-executable instructions which, when executed, cause a computer system to simulate interaction between a surgical tool and biological tissue according to a method comprising: receiving tool input device information representative of movement of a physical representation of a portion of a simulated surgical tool; simulating, based on the tool input device information, an interaction between the simulated surgical tool and a simulated biological tissue, the simulating comprising using a plurality of simultaneously running computational threads for calculating the mechanics of the simulated biological tissue, at least some of the threads providing their calculations to one or more associated threads for use in such associated threads' calculations, wherein the plurality of threads comprise at least two threads that have different spatial resolutions including a first spatial resolution and a second spatial resolution lower than the first resolution, wherein the first higher spatial resolution is utilised in respect of an area where a user is interacting; and displaying on a display a visual representation of the simulated interaction between the simulated surgical tool and the simulated biological tissue, the visual representation being based on the calculations of the plurality of threads.

According to a third aspect of the present invention there is provided a biological tissue simulator comprising: a physical representation of a portion of a simulated surgical tool; a tool input device constructed and arranged to measure movement of the physical representation; a display; and a computer program operatively connected to the tool input device and the display, wherein the computer program is programmed to simulate an interaction between the represented surgical tool and a simulated biological tissue, the computer program comprising a plurality of computational threads for calculating the mechanics of the simulated biological tissue, the plurality of computational threads being programmed to run simultaneously, at least some of the threads being programmed to provide their calculations to associated threads for use in such associated threads' calculations, wherein the plurality of threads comprise at least two threads that have different spatial resolutions including a first spatial resolution and a second spatial resolution lower than the first resolution, wherein the first higher spatial resolution is utilised in respect of an area where a user is interacting; wherein the computer program is programmed to receive from the tool input device information relating to the measured movement and provide the tool input device information to one or more of the threads, and wherein the computer program is programmed to provide to the display for displaying on the display a visual representation of the simulated interaction between the simulated surgical tool and the simulated biological tissue, the visual representation being based on the calculations of one or more of the threads.

There is disclosed an open surgical training system capable of producing an experience similar to what the student/user would have were the student operating on a live patient, with realistic hands-on experiences for a wide variety of conditions and procedures, while using objective, quantitative metrics developed by experienced surgeon-educators to assess a student's actual performance and skill level. This approach is intended to result over time in better-trained surgeons exposed to a wider variety of cases and situations than is presently possible, reduced patient risk, better utilization of scarce training resources, and fewer surgical errors.

There is a need for a surgical simulator that simulates interaction between tools (e.g., forceps, scalpel, clamps, scissors, pick-up, needle holder, needles, etc.) and biological tissue (e.g., skin, underlying tissue, ligaments) with more accurate real-time haptic and/or visual feedback. One or more embodiments of the present invention improves the accuracy and real-time speed of such feedback.

One goal of simulation-based surgical training is to create a user experience that replicates actual surgery with many or all the interaction characteristics between the student and the body that are helpful to teach the student the connections between the student's actions and the body's response. Since in general it is not known what are exactly those connections that inform the mind to produce effective surgical skills, an alternative goal is to reproduce actual surgery as nearly as possible in the simulation. Specific aspects of this experience can then be controlled to provide a greater diversity of situations to a user than the user would experience in typical training situations, while doing so with greater patient safety. A challenge in providing this experience is creating a high-fidelity simulation of surgical tools interacting with tissue. The fidelity has a variety of components which each are preferably of high enough resolution to achieve the overall effect. One component is the resolution of the model of the physical world (anatomy, tools, environment). Another component is the computation of changes and interactions in the model. A third component is the presentation of the results of the dynamic model to the user. These and/or other components present problems to be solved, separately or in combination.

It is preferable for human visual systems to be stimulated (presented with images) at 30 Hz or greater in order for them to perceive a continuous display. While lower frequencies are within the scope of the present invention, the use of such lower frequencies may impair the continuity of the display. The human touch system is preferably stimulated (e.g., through haptic rendering) at rates greater than 1 KHz for a continuous perception of touch. However, it is within the scope of the present invention to make any suitable trade-off or compromise between the calculation and visualization frequencies, the haptic feedback frequencies, and available and/or cost-effective processing and haptic feedback power/speed.

While systems exist that provide haptic and visual feedback at these rates, the quality of the experience is typically limited. These simulators do not provide enough capability to be sufficiently close to actual surgery to make the simulation believable/useful to the user. Current computer hardware and software technology has advanced to the point where achieving a high-resolution 30 Hz or better visual display of a surgical model is quite possible. Providing the same level of believability for haptic feedback and having the physics-based surgical scene model respond to grasping and cutting with similar fidelity is not currently achievable through conventional simulators.

There is provided a meaningful simulation experience of tissue grasping.

One or more examples described herein increase the fidelity/resolution available in a surgical simulator at the site where the user or tool is interacting with the simulated tissue. This approach can be used to train a wide range of surgical activities from generalizable basic skills such as suturing to more complex complete surgeries that include activities specific to the procedure being taught. It could also be extended to mechanical and manufacturing processes that involve heterogeneous deformable materials.

There is disclosed a method for simulating interaction between a surgical tool and biological tissue. The method includes receiving tool input device information representative of movement of a physical representation of a portion of a simulated surgical tool. The method also includes simulating, based on the tool input device information, an interaction between the simulated surgical tool and a simulated biological tissue. Simulation includes using a plurality of simultaneously running computational threads for calculating the mechanics of the simulated biological tissue. At least some of the threads provide their calculations to one or more associated threads for use in such associated threads' calculations. The method also includes displaying on a display a visual representation of the simulated interaction between the simulated surgical tool and the simulated biological tissue. The visual representation is based on the calculations of the plurality of threads.

There is disclosed a method in which at least some of the threads provide their calculations to their associated threads asynchronously.

There is disclosed a method in which plurality of threads include at least two threads that have different resolutions (e.g., temporal, spatial, etc.).

There is disclosed a method in which using the plurality of threads includes using a moving thread whose spatial coverage on the simulated biological tissue moves in response to simulated movement of the simulated surgical tool. Using the plurality of threads may also include using a fixed-position thread whose spatial coverage on the simulated biological tissue does not move in response to the simulated movement of the simulated surgical tool. The fixed-position thread may have a lower computational resolution than the moving thread. Lower computational resolution may include temporally lower computational speed and/or spatially lower computational resolution. Using the moving thread may include moving the spatial coverage of the moving thread so as to maintain coverage of a moving location of interaction between the simulated surgical tool and the biological tissue.

There is disclosed a method in which the plurality of threads include at least one selectively activated thread. The method further includes activating and deactivating operation of the at least one selectively activated thread based on a determination of whether the simulated surgical tool's interaction with the simulated biological tissue would impact the calculations of the at least one selectively activated thread by more than a predetermined threshold.

There is disclosed a method in which simulating the interaction includes determining a simulated haptic response based on the calculations of one or more of the threads. The method further includes converting the simulated haptic response into a physical haptic response that is applied to the physical representation. The physical haptic feedback may include a load applied to the physical representation. According to one or more of these embodiments, the method may also include providing the physical haptic response to the physical representation at a higher frequency than the simulation and/or visual representation.

There is disclosed a method in which simulating includes running at least two of the plurality of threads on different computer cores from each other.

There is disclosed a method in which simulating includes running at least one of the plurality of threads on a graphics processing unit.

There is disclosed a method in which using the plurality of computational threads includes using a first layer thread to calculate changes to a first simulated layer of the biological tissue, using a second layer thread to calculate changes to a second simulated layer of the biological tissue that adjoins the first simulated layer, and linking the first and second layer threads to each other so that their adjacent boundaries conform to each other. There is disclosed a method in which the first simulated layer includes a simulated surface layer, and the second simulated layer includes a simulated portion of the biological tissue below the simulated surface layer. There is disclosed a method in which the first and second layer threads each comprise numerical tissue mechanics computational threads. There is disclosed a method in which using the plurality of computational threads includes operating the first layer thread at a higher resolution than the second layer thread.

There is disclosed a method in which the represented surgical tool includes a grasping tool. Simulating includes simulating a grasping by the tool of a portion of the simulated biological tissue.

There is disclosed a method in which displaying includes displaying on the display simulated consequences of movement of the physical representation on the simulated interaction within 150 ms.

There is disclosed a method in which the method also includes providing haptic feedback to the physical representation of simulated consequences of movement of the physical representation on the simulated interaction within 150 ms.

There is disclosed a method in which the method further includes calculating a viability of a portion of the simulated surgical tissue based on the simulated interaction. The method may also include visually displaying the calculated viability as part of the visual representation. The simulating may include simulating an effect of the simulated interaction on a cellular structure of the simulated biological tissue. The calculating of the viability may include calculating the viability as a function of said effect. The calculating of the viability may include calculating of the viability as a function of blood flow through the simulated biological tissue.

There is provided a machine-readable medium encoded with machine-executable instructions which, when executed, cause a computer system to simulate interaction between a surgical tool and biological tissue according to one or more of the above-discussed methods according to various embodiments of the present invention. The method may include receiving tool input device information representative of movement of a physical representation of a portion of a simulated surgical tool; simulating, based on the tool input device information, an interaction between the simulated surgical tool and a simulated biological tissue, the simulating comprising using a plurality of simultaneously running computational threads for calculating the mechanics of the simulated biological tissue, at least some of the threads providing their calculations to one or more associated threads for use in such associated threads' calculations; and displaying on a display a visual representation of the simulated interaction between the simulated surgical tool and the simulated biological tissue, the visual representation being based on the calculations of the plurality of threads.

There is provided a biological tissue simulator that includes a physical representation of a portion of a simulated surgical tool; a tool input device constructed and arranged to measure movement of the physical representation; a display; and a computer program operatively connected to the tool input device and the display. The computer program is programmed to simulate an interaction between the represented surgical tool and a simulated biological tissue. The computer program includes a plurality of computational threads for calculating the mechanics of the simulated biological tissue. The plurality of computational threads are programmed to run simultaneously. At least some of the threads are programmed to provide their calculations to associated threads for use in such associated threads' calculations. The computer program is programmed to receive from the tool input device information relating to the measured movement and provide the tool input device information to one or more of the threads. The computer program is programmed to provide to the display for displaying on the display a visual representation of the simulated interaction between the simulated surgical tool and the simulated biological tissue. The visual representation is based on the calculations of one or more of the threads.

There is disclosed a method in which the threads are programmed to provide their calculations to their respective at least one interrelated threads asynchronously.

There is disclosed a method in which the plurality of threads include at least one selectively activated thread. The computer program may be programmed to activate and deactivate operation of the at least one selectively activated thread based on a determination of whether the simulated surgical tool's interaction with the simulated biological tissue would impact the values computed by the at least one selectively activated thread by more than a predetermined threshold.

There is disclosed a method in which the simulator further includes a haptic feedback device operatively connected to the physical representation and computer program. The computer program may be programmed to determine a simulated haptic response based on the calculations of one or more of the threads and provide the simulated haptic response to the haptic feedback device. The haptic feedback device may be constructed and arranged to convert the simulated haptic response into a physical haptic feedback applied to the physical representation.

There is disclosed a method in which the simulator includes a plurality of computer cores or graphics processing units. The computer program is programmed to run at least two of the plurality of threads on different cores from each other.

There is disclosed a method in which the plurality of computational threads includes a surface layer thread that calculates changes to a simulated surface of the biological tissue, and an inner layer thread that calculates changes to a simulated portion of the biological tissue below the simulated surface. The surface and sub-surface layer threads may be linked to each other so that their adjacent boundaries conform to each other. The surface and sub-surface layer threads may each include spatially discretized computations threads. The computer program may be programmed to run the surface layer thread at a higher resolution than the sub-surface layer thread.

There is disclosed a method in which the simulator is constructed and arranged to display on the display simulated consequences of movement of the physical representation on the simulated interaction within 1 second.

There is disclosed a method in which the simulator is constructed and arranged to provide haptic feedback to the physical representation of simulated consequences of movement of the physical representation on the simulated interaction within 1 second.

There is disclosed a method in which the computer program is programmed to calculate a viability of a portion of the simulated surgical tissue based on the simulated interaction. The computer program may be programmed to provide to the display for displaying on the display a visual representation of the calculated viability. The computer program may be programmed to simulate an effect of the simulated interaction on a cellular structure of the simulated biological tissue, and calculate the viability as a function of said effect. The computer program may be programmed to calculate the viability as a function of blood flow through the simulated biological tissue.

Additional and/or alternative objects, features, aspects, and advantages of the present invention will become apparent from the following description, the accompanying drawings, and the appended claims.
WO-A-2007/019546 discloses a wound debridement simulator for simulating procedures relating to surgical debridement of the wound. The wound debridement simulator includes a visual display for displaying a simulated rendering of a portion of a human body upon which a wound has been inflicted. The wound debridement simulator also includes a modelling system in communication with the visual display, the system causing the animated rendering to change in response to a simulated touching of the portion of the human body.

### BRIEF DESCRIPTION OF THE DRAWINGS

For a better understanding of embodiments of the present invention as well as other objects and further features thereof, reference is made to the following description which is to be used in conjunction with the accompanying drawings, where:
FIG. 1 is a perspective view of an open surgery simulator 1 according to an embodiment of the present invention;
FIG. 2 is a perspective view of simulated biological tissue for use with the simulator of FIG. 1;
FIG. 3 is a perspective view of simulated interaction between simulated biological tissue and simulated surgical instruments;
FIG. 4 is a perspective view of a simulation of biological tissue in which the piercing of a blood vessel is causing blood accumulation within an incision;
FIG. 5 is a diagrammatic flowchart of interactions between various inputs, outputs, and computational threads of a biological tissue simulator according to an embodiment of the present invention; and
FIG. 6 is an illustration of the visual representation of a simulation according to an embodiment of the present invention.

### DETAILED DESCRIPTION OF EXEMPLARY EMBODIMENTS OF THE INVENTION

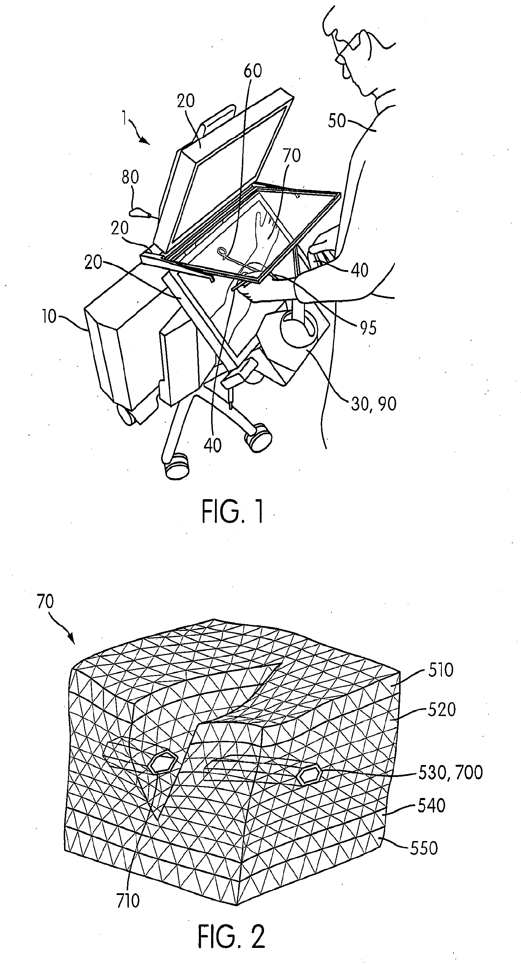

FIG. 1 illustrates an open surgery simulator 1 according to an embodiment of the present invention. The simulator 1 comprises a processor 10, a simulation program 15, a display 20, a tool input device 30, a physical representation of a surgical tool 40, a stand 80, and a haptic output device 90.

The processor 10 may comprise any suitable processor for running the simulation program 15. For example, the processor 10 may comprise a conventional personal computer, or may alternatively comprise a processor specifically built for quickly performing the numerous calculations involved in the simulation program 15. The processor 10 may include a one or more graphics processing units (GPUs). The processor 10 may include multiple cores for simultaneously processing a variety of computational threads associated with the simulation. According to one embodiment, the processor 10 comprises two dual-core processors using a dual SLI graphics card system and an Ageia PhysX physics processor unit (PPU). The use of multiple processing cores and/or CPUs and/or PPUs and/or GPUS may give greater computational resolution/fidelity (graphical, temporal, etc.) to the tissue simulation within the constraints of available computational power.

The simulation program 15 runs on the processor 10, and may be in any suitable form (e.g., program on a hard drive, CD, DVD, network server, etc., machine-readable medium encoded with machine-executable instructions executable by the processor 10) and format for a particular associated processor 10 (e.g., machine-readable medium for use on a processor 10 running DOS, WINDOWS, LINUX, a MAC OS, etc.). The program 15 is encoded with and designed to simulate the interaction between one or more simulated surgical tools 60 and simulated biological tissue 70 (a human forearm in the embodiment illustrated in FIGS. 1-4). Among other things, the program 15 (a) receives inputs via the processor 10 and tool input device 30, (b) uses those inputs to calculate changes to the simulated tools 60 and tissue 70, (c) converts those results into information representing a visual representation of the simulated interaction between the simulated surgical tool(s) 60 and the simulated biological tissue 70, and information representing haptic feedback to apply to the tool(s) 60, and (d) outputs such information, via the processor 10, to the display 20 and haptic output device 90. Various algorithms used by the program 15 will be discussed in greater detail following a discussion of various remaining components of the simulator 1.

The display 20 operatively connects to the processor 10 to receive information representing a visual representation of the simulated interaction between the simulated surgical tool(s) 60 and the simulated biological tissue 70, as calculated by the processor 10 and program 15. Consequently, the display 20 provides a visual representation of the simulation based on the calculations of the processor 10 and program 15.

The display 20 may comprise any type of suitable display (e.g., LCD monitor(s), television(s), CRT monitor(s), glasses/goggles with incorporated displays (such as used in connection with VR technology, etc.). The illustrated display 20 comprises two LCD monitors that provide a stereoscopic output that provides a user with a three-dimensional simulation, though the display 20 may provide a two-dimensional simulation without deviating from the scope of the present invention. The processor 10 and program 15 used with such a 3-D embodiment calculates a stereoscopic representation and outputs it to the display 20 to provide a stereoscopic visual representation of the simulated interaction between the simulated surgical tool 60 and the simulated biological tissue 70. The display 20 may provide such stereoscopic information using any suitable mechanism (e.g., a planar wide-screen passive stereoscopic monitor (shown in FIG. 1), offset polarization of LCD displays along with offset polarized glasses on the user, color-shifted display with color-divergent glasses on the user, autostereoscopic enabled displays incorporating specialized image deflection lenses).

The display 20 and/or another part of the simulator 1 may incorporate a device (e.g., motion/position/orientation sensing helmet or glasses) for tracking the head of the user. The program 15 may then adjust the visual representation output to the display 20 to account for the changed position and direction of the user's head such that the user can change their viewing position and orientation in a realistic and intuitive manner.

The tool input device 30 operatively connects to the processor 10 and physical representation 40 of the surgical tool 60 to relay to the processor 10 tool input device information representative of movement and/or force applied by a user to the representation 40. The tool input device 30 may relay a variety of motion inputs (e.g., translation in one to three orthogonal directions, rotation along one to three orthogonal axes, and/or tool-specific motion such as opening and closing of forceps or rotation of a surgical drill bit) and/or a variety of force inputs (e.g., translational force in one to three orthogonal directions, rotational moment/torque about one to three orthogonal axes, and/or tool-specific forces such as opening/closing force applied to a forceps or rotational torque applied to a surgical drill bit).

The haptic output device 90 converts simulated forces/moments/torques applied to the simulated tool 60, as calculated by the processor 10 and program 15, into real loads (e.g., forces/moments/torques) applied to the representation 40. The haptic output device 90 may create such loads along any one or more of the degrees of freedom of the tool input device 30 (e.g., 3 translational directions, 3 rotational directions, tool-specific directions). The program 15 may provide information representative of haptic feedback to the haptic output device 90 through a multi-rate approach that allows effective feel to be provided to the user independent of the varying computational load on the processor 10. Alternatively, the simulator 1 may omit such haptic feedback without deviating from the scope of the present invention.

According to one or more embodiments of the present invention, the tool input device 30 and haptic output device 90 are integrated. In the illustrated embodiment, each integrated tool input device 30 and haptic output device 90 comprises a SensAble Omni haptics devices connected to a respective representation 40 of a simulated tool 60. Alternatively, each integrated input/output device 30, 90 may comprise any other suitable device (e.g., a Falcon device made by Novint Technologies, Inc.).

The physical representation 40 of each simulated surgical tool 60 may comprise any type of suitable physical representation 40 of the simulated tool 60. The representation 40 may comprise the actual, physical surgical tool being simulated. Alternatively, the representation 40 may comprise just a portion of the actual, physical tool that interacts with a user. Alternatively, the representation 40 may comprise a generic representation (e.g., a dowel that represents a handle of a scalpel, etc.). The physical representation may connect to the tool input device 30 and haptic output device 90 using any suitable interface that transfers force, motion, and/or resistance therebetween along the degrees of freedom being input/output.

According to one or more embodiments, a tool mount 95 is attached to an end effector of the tool input device 30. The tool mount 95 enables easy releasable/exchangeable attachment of a variety of actual surgical tools. The tool mount 95 may comprise any suitable mechanism that transfers force/torque and/or motion between the representation/actual tool 40 and the tool input device 30 and haptic output device 90.

For example, in the case of a forceps, the tool input device 30 may comprise an angle encoder for inputting a relative open/close position of the two pivotally attached sides of the forceps. Conversely, the haptic output device 90 may comprise a variable resistor to such pivotal motion of the representation 40 of the forceps (e.g., via a motor, variable magnet-based resistance, solenoid-actuated, variable resistance clutch between the sides of the forceps, etc.).

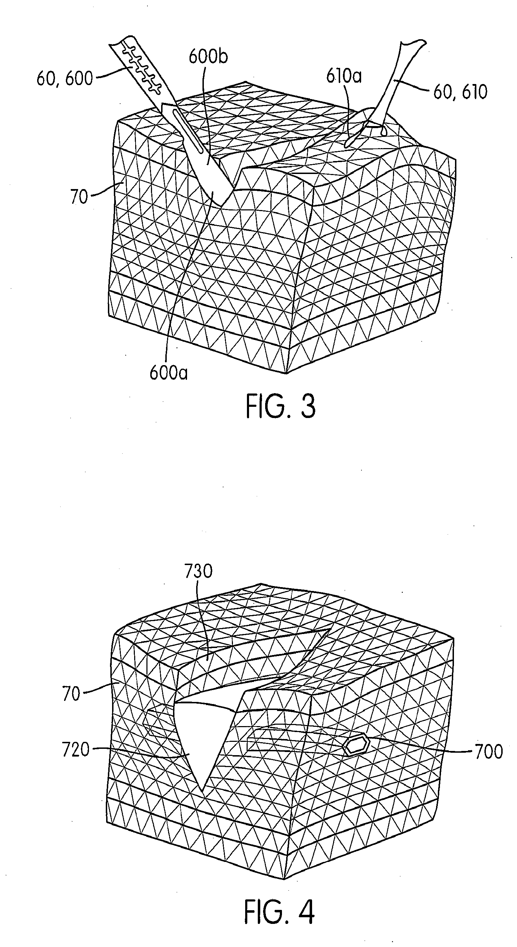

In the illustrated embodiment, two representations 40 of two simulated surgical tools 60 are provided. For example, as shown in FIG. 3, both a scalpel tool 60, 600 and a grasping tool 60, 610 may simultaneously be simulated. Accordingly, a tool input device 30 and haptic output device 90 is provided for each representation 40 of a tool 60. The range of movement and interaction between the hands, representations 40, tool input devices 30, and haptic output devices 90 allows two-handed surgery to be simulated. However, greater or fewer tool input devices 30, haptic output devices 90, and associated representations 40 may be used without deviation from the present invention (e.g., a single simulated tool 60, three tools 60, etc.). In the illustrated embodiment, the tool input devices 30 and display 20 provide a work volume of approximately 15 cm by 15 cm by 15 cm sufficient for focused two handed tissue manipulation such as wound closure.

As shown in FIG. 1, a portion of the display 20 is interposed between the user's eyes and the physical representations 40 of the tools 60 so that the user sees the visual representations of the display 20 when looking in the direction of the physical representations 40 of the tools 60. The user 50 stands as a surgeon would in an operating room, moves the user's hands and representations 40 as if the user were performing surgery, and sees the visual representation of the display 20. Consequently, when the user 50 looks toward the representation 40, the user will see the simulation 60 thereof in its place. The degree to which this representation supersedes the image of the user's hand holding the surgical tool underneath the half silvered mirror display surface is controlled by a light attached under display surface which can be controlled by the simulation such that when the light intensity is increased the user's hands with the tools are more visible and when the light intensity is decreased, the user sees the computer generated representation of the surgical tools instead.

In the illustrated embodiment, the tool input device 30, haptic output device 90 and display 20 are mounted to a height-adjustment stand 80 to support a broad range of individuals standing at its simulated operating table. The illustrated height-adjustment stand 80 comprises a lever-actuated spring/piston assembly, as is conventionally used in adjustable chairs. However, any other suitable height-adjustment mechanism may alternatively be used without deviating from the scope of the present invention. Moreover, the height-adjustment mechanism may be omitted altogether without deviating from the scope of the present invention.

According to one or more embodiments, the simulator 1 has a physical configuration that is compact enough to be easily moved, is height-adjustable to support a broad range of individuals standing at its simulated operating table, and enables users be able to hold the actual surgical tools (or physical representations 40 thereof) they will use in real life.

Hereinafter, the manner in which the processor 10 and program 15 simulate the interaction between a simulated surgical tool 60 and simulated biological tissue 70 according to various embodiments is described in greater detail. To improve the simulation, it is advantageous to more accurately and/or quickly simulate interactions with biological tissue 70. While there are significant individual variations in skin behavior, the overall behavior is nonlinear and time-dependent. This has implications relative to the computational model that is chosen to model tissue structural behavior.

The prior art does little to actually address the specific mechanics of the interactions between tissue and geometry and materials of surgical instruments in a simulation. Typically force characteristics that are not reflective of the physical situation in real-life have been employed in simulation to accomplish tissue manipulation such as grasping. For example, when the grasping end of the tool passed within a certain proximity of a graspable tissue with a specified jaw opening while it was in the process of closing, the tool will suddenly latch-on to the tissue. It goes without saying that this results in a poor training experience for a student where the primary learning objectives include grasping sutures, and tissue.

Skin (and human soft tissue in general) is viscoelastic. Thus, it has complex mechanical properties reflecting its inhomogeneous solid-fluid composition including the elastic properties of solid materials and the viscous properties of fluids. The elastic properties of the skin relate to the changes that occur when force is applied to the skin, such as from a tool or the surrounding environment, combined with any internal pre-strain in the tissue. They govern the skin's ability to deform, i.e. to stretch, contract and compress. The viscous characteristics of the skin relate to, among other things, the delayed changes occurring after time: the decrease in stress over time when a constant strain is applied (the stress relaxation effect) and the increase in length over time when a constant strain is applied (the creeping effect). For the purposes of simulating grasping, the viscous characteristics appear to have a negligible influence on user perception and as such are not part of the current model, but may be incorporated into the model without deviation from the present invention. Given the current state of computational power, inclusion of such effects would have a negative impact on real-time performance. However, the processor 10 may include such effects without deviating from the present invention, especially if processor power/speed is sufficiently high. Inclusion of such viscous property is advantageous (though not required by the present invention) as simulations of longer duration are accomplished and the viscous effects come more into play in user experience and results.

Three separate but interrelated issues are preferably addressed to create a computationally appropriate simulation of tool-tissue interaction according to one or more embodiments of the invention. First, the simulation preferably incorporates a biomechanics-appropriate constitutive model of the tissue. Next, an engineering mechanics representation of the mechanical stress-strain within a structure composed of the constitutive tissue model is preferably developed and implemented in code. Finally, the process of detecting and computing the response to the tool contacting tissue preferably involves its own separate treatment.

Skin tissue is primarily composed of collagen fibril and elastin constituents. Together these account for the strength and the elasticity of skin. Depending on the nature of the tissue being modeled either an isotropic or anisotropic model of the tissue elasticity could be built from these fundamental building blocks. A model that takes into account this level of detail is desirable from a tissue reaction standpoint, but such desirability must be weighed against the cost/feasibility using available computing capability. According to one or more embodiments of the present invention, in substitute for such a model, a multi-layered approach to tissue modeling may be used.

FIG. 2 is a perspective visual representation of simulated tissue 70 generated by the simulator 1, using a multi-layered approach to tissue modeling. As shown in FIG. 2, the simulated tissue 70 (which, as illustrated, comprises a portion of a patient's arm) comprises a skin layer 510, an adipose tissue layer 520, a vein layer 530, a connective tissue layer 540, and a muscle layer 550. However, a variety of additional and/or alternative layers may be used depending on the goal of the particular simulation and the type of tissue being simulated (e.g., several layers for the outer skin surface to represent epidermis behavior relative to dermis, one or more layers for ligaments, bone, body organs, various additional blood vessels, etc.).

In such a multi-layered approach, the layers may be implemented as one or more of a number of engineering mechanics derived implementations such as tetrahedrally-based, nonlinear, hyperelastic finite element tissue layers coupled to an outer surface layer that could use computations reflective of the physics of cloth or a thin layer of material. It also may be composed solely of several tetrahedrally-based finite element layers. FIG. 2 illustrates an example finite element arrangement of elements in each layer 510, 520, 530, 540, 550. The dermis/adipose/bulk layer 520 captures the bulk properties of the tissue and provides a means for accurately simulating real tissue interactions in real-time. The surface/skin layer 510 is layered on top of the bulk layer 520. The surface layer 510 is capable of operating at a different (higher) resolution than the bulk layer 520. Higher resolution can comprise higher spatial resolution (e.g., discrete element size is smaller) and/or higher temporal resolution (faster computational rate). This surface layer 510 operates with its own collision detection and response behavior though it is coupled to the underlying layer 520 to ensure that its overall behavior appropriately reflects the bulk behavior of the tissue 70. This coupling can be a distribution of simple spring-dampers or it can be complex actively controlled set of actuators that achieve a specific goal such as smoothing out behavioral discontinuities between the underlying and surface layers. In turn, the remaining adjoining layers 520/530, 530/540, 540/550 are similarly coupled to each other. The resolution of this surface layer 510 (and/or other layers) can vary both spatially and temporally. Thus it can deliver a high-resolution patch to exactly where it is needed to calculate a detailed response to interaction with a tool 60. By concentrating resolution (which equates to computational resources) in this manner the skin reaction to the tool 60 and the grasping can be more reflective of actual skin behavior, and can be done on a more real-time computational basis. The inners layers 520, 530, 540, 550 are linked to the surface layer 510 so that they conform to the behavior imposed by the surface layer 510 and conversely the surface layer 510 conforms to the underlying tissue behavior as determined by the remaining layers 520, 530, 540, 550. This computational topology frees the surface layer 510 to use computational approaches favoring speed over accuracy that allow it to run at a higher update rate than the underlying layers 520, 530, 540, 550 which in turn favor accuracy over speed in their calculations. The result according to one or more embodiments of the present invention is that the surface layer 510 has sufficient computational capacity to include response to frictional forces as it interacts with the tool and to check for, and respond to, self-intersections in real time.

The layers 510, 520, 530, 540, 550 incorporate the physical characteristics/properties/composition of the portion of tissue 70 being modeled. Such characteristics/properties are readily available from existing sources such as the published biomechanics literature as well as through traditional materials testing machines. The illustrated tissue 70 comprises a portion of human arm tissue. However, any other tissue portion (or other material) may alternatively be modeled, as desired for use with the simulator 1, depending on the desired simulation. For example, the simulator 1 may include various basic simulation scenarios (e.g., making a particular incision into a human forearm (or other body part), manipulating skin tissue in a human leg, manipulating a wound on any part of a body, suture placement, needle and suture handling, etc.) as well as more complex scenarios (e.g., wound and tissue handling, hemostasis, skin and tissue debridement, excision, suturing a wound, know tying, lesion excision, tube anastomosis, etc.). The initial simulated tissue 70 may include existing wounds, cuts, etc. In one embodiment, the simulator comprises five basic suture pattern scenarios and five knot types, based on analyzing definitive references on suture training from such groups as the Association of Program Directors of Surgery, the American College of Surgeons, and the Royal College of Surgeons. These high-level teaching objectives were vetted with a number of experienced surgical instructors.

According to one or more embodiments of the present invention, the simulator 1 provides a significant step forward in surgical simulation capability. By using a layered computation topology, it is possible, according to one or more embodiments of the invention, to model and simulate real-world behaviors of grasping. When grasped, skin bunches due to frictional contact between the tool 60 (e.g., forceps, tweezers, other clamps) and tissue 70, which affects the how the tool 60 grasps the skin tissue 70 and therefore modifies the nature of how the tool 60 holds the skin tissue 70. Likewise, depending upon the frictional response, the tool 60 may not completely grasp the skin tissue 70 and it will slip out of its grasp. In addition, the local magnitude of the tissue stress within or beneath the jaws of the grasping surgical tool 60 can be computed and the effect of the loading upon the viability of the tissue 70 can be recorded and displayed. Mechanical stress over time has a significant effect upon tissue health as the result of a number of effects such as direct disruption of cellular structure as well as restriction of blood flow and subsequent cellular death. Being able to compute and show this provides a significant, and unavailable in real life, benefit to users in allowing them to learn the implications of their tissue manipulation actions upon the long term outcome/health of the tissue 70 they have affected. These complex responses are beneficial to learning effective tissue manipulation that provides good tissue control while respecting the mechanical integrity and viability of the tissue 70. Such an experience provides training in the fine motor skills necessary to carry out the subtleties of various procedures without unnecessarily damaging the tissue 70. By moving to this level of fidelity of simulation, one or more embodiments of the present invention make it possible to train both high-level cognitive skills and fine-grained motor skills through simulation.

FIG. 3 is a perspective visual representation of the interaction between two tools 60 (a scalpel 600 and a grasping tool 610) and tissue 70.

The processor 10 and program 15 may obtain the high-fidelity simulation of interaction with tissue 70 via a multi-threaded approach to computing changes to the physical world (i.e., the tissue 70 and tools 60) via the simulation. As is presently done in computer gaming applications, the processor 10 and program 15 may use multiple cores and/or CPUs and/or GPUs to synchronously update between higher and lower resolution models of the physical world (e.g., biological tissue 70). Alternatively, one or more embodiments may provide for asynchronous updating by various threads. When operating asynchronously, the different threads need not operate in reference to a master clock, but instead can provide their results to other threads as they are calculated. Other threads may choose to use or not use such results (e.g., not using such results in the thread that is already midway through a computation based on previous results from the other threads).

According to one or more embodiments, the multi-threaded approach is based on the Real Time Innovations (RTI) Constellation real-time system computer-aided software engineering tool. The Constellation software has been used in a variety of commercially deployed complex distributed hardware/software systems.

According to various embodiments, there may be two or more levels of fidelity that can be defined by spatial, temporal, or other measures. For example, each processing thread operates on a model of the physical world, where the models may be at the same resolution for multiple threads or each thread may have its own resolution. Each thread computes changes to its computational representation of the model based on input from any user input device (mouse positioning, information received from the tool input device 30, etc.) or computations from other threads. Thus as a thread computes changes to its model it can make those changes available for other threads by all the threads sharing common memory pools where results are easily accessible amongst the threads. In this way, a modest-fidelity but fast thread computing surface responses to tissue manipulation, such as the skin/surface layer 510, can be coupled to a high-fidelity, slower thread(s) computing the underlying tissue response (e.g., the layers 520, 530, 540, 550) such that the underlying tissue behavior is used to correct the surface behavior through the interconnection that transfer forces and/or movements of the respective layers to one another. Either of these threads may make use of more than one computational unit, such as a CPU core or GPU, to further speed their computations through parallelizing the process.

Task assignment can either be done *a priori* or as an active thread. In one exemplary *a priori* case, a number of threads are established (taking into account the processing power of the current hardware configuration). These threads are given fixed responsibilities for computing model changes. Typically, some threads are assigned to maintaining overall fidelity of the model from a physics standpoint while others are given the task of ensuring that the rendering threads have a continuous supply of information that is preferably sufficiently close to the high-resolution model that the user's experience remains relatively true to an actual surgery (to the extent possible or desired). In a computationally rich environment there may also be threads that help to bridge the gap between the highest- and lowest-resolution aspects. For example, a thread may be used to smoothly distribute the movement of a more spatially course soft tissue layer across a spatially finer overlying tissue layer and vice versa or provide temporal blending between two entities running at different update rates such that the slower thread provides smoothly changing behavior (such as changes in movement or forces) to the faster thread as could happen in connecting a simulation thread to a haptic thread.

Keeping the entire model up-to-date with exact physics is generally too computationally expensive to run at an interactive rate. The multi-threaded approach is one portion of an exemplary solution to this problem according to an embodiment of the invention. A second aspect of one or more embodiments comes in with the choice of task assignment. The various threads can be assigned not just differing resolutions at which to calculate, they may also be given different spatial locations to compute. These locations may or may not overlap. By consideration of current user inputs, the spatial location that is assigned to a task may change over the course of processing. By changing the spatial focus of the modeling thread(s) to concentrate on areas where the user is interacting, a localized high-resolution spot may be maintained exactly where needed in order to present the user with a more accurate surgical experience while simultaneously keeping the overall model in a consistent state.

It should be noted that the task assignment is not limited to moving a high-resolution thread to the area where the user is interacting. It may be the case, due to interconnections between anatomic entities within the model, that there are additional locations or structures not near the interaction site that require additional attention. Consider the case of the user grasping a ligament. To make the simulation more accurate, a high-resolution spatial thread could cover the area near the grasping input, while a second high resolution spatial thread could cover the significant area near the attachment points of the ligament.

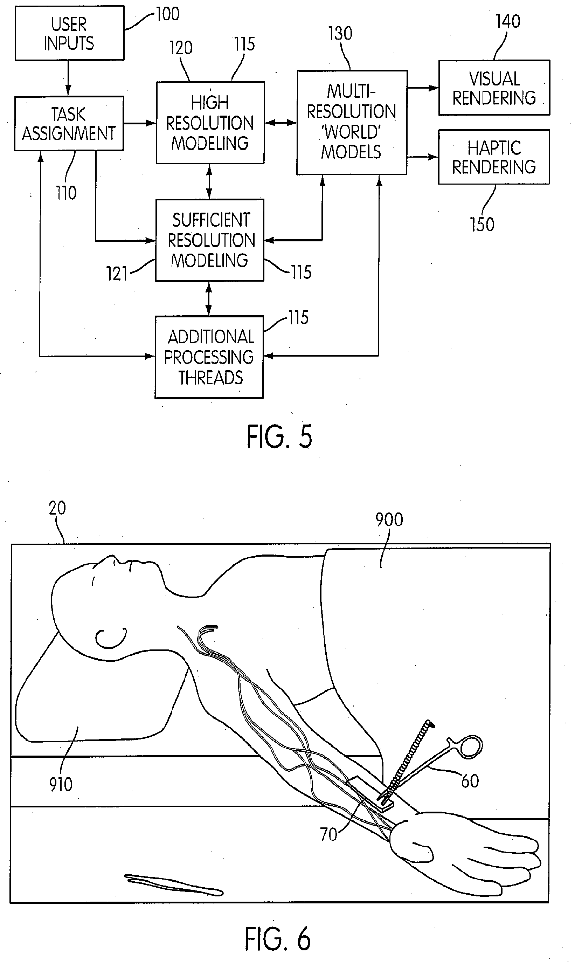

FIG. 5 illustrates a flowchart of the thread-based operation of the program 15. Each thread running on the program 15 and processor 10 may have one or more roles in providing the simulation. An input thread 100 captures user inputs from input devices such as the tool input devices 30 and provides the captured information to a task assignment thread 110. The task assignment thread 110 assigns appropriate resulting tasks to processing threads 115 such as a high resolution modeling thread 120 (e.g., a high resolution finite element model running a multi-physics code called PhysBam to produce accurate simulation of soft tissue behavior in response to its being manipulated by surgical tools) and a sufficient resolution modeling thread 121 (e.g., a modest accuracy but fast and more visual detailed surface deformation model running the Nividia hardware accelerated PhysX). The unique numerical capabilities of the PhysBam code have been widely published in SIGGRAPH and other peer reviewed journals, and PhysBAM provides physics modeling for various complex Hollywood special effects animations that seek to reflect real life behavior. A skin simulation thread 115 may include code that implements a nonhomogeneous, nonlinear, hyperelastic finite element representation to produce engineering design and analysis-quality results. This code uses an iterative solver that is amenable to parallelization across multiple processors. According to one embodiment, the skin simulation thread 115 is tuned to provide real-time response to user manipulation of tissue 70.

In one or more embodiments, PhysBAM is not suitable for simulating either sutures or fluids in real-time. In such embodiments, other physics-based code is used, such as formulations running on Nvidia PhysX GPU acceleration as well as tailor-made physics code running on multiple processor cores, either CPU or GPU-based. These custom codes are typically derived from physics-based numerical methods to provide fast behavior that sufficiently reflective of perceived behavior of entities interacting with one another in a simulation. The suture simulation may utilize a Cosserat elasticity formulation, or one of a number of other approaches that also take into account the unique physical properties of thin elastic members, running asynchronously with the PhysBAM soft tissue simulation as well as the PhysX-based computations and communicating with them via shared constraint forces and moments which can consist of either passively computed constraints or actively computed interactions such as occur with linear complementarity problems (LCP) traditionally used in interactive rigid body dynamics simulations present in computer games.

The processing threads 115 use the inputs 100 to compute changes to the simulated tool(s) 60 and tissue 70 (e.g., position changes, deformation, cutting, and/or tearing of the tissue 70, etc.). As the processing threads 115 compute changes, they provide their results to each other, to the task assignment thread 110, to a visual rendering thread 140, and to a haptic rendering thread 150. The thread 121 ensures that the haptic information is smoothly varying. The thread 120 ensures that the model changes are shown with a sufficient reflection of physical reality.

The visual rendering thread 140 converts the results of the processing threads 115 into information representative of a visual representation of the tool(s) 60 and tissue 70, and provides this information to the display 20. Such rendering may include rendering of the skin surface, a surface of internal organs/tissue, and/or a surface created by a scalpel's incision.

The illustrations in FIGS. 2-4 are examples of the visual representations that could be rendered by the thread 140. However, the visual representations need not show FEA elements, and may or may not provide a cross-sectional view. For example, the visual representation of the simulated tissue 70 may show just a combination of one or more of the skin surface, the surface of any incisions/cuts into the skin, the surgical tools 60, and blood (e.g., the portions of the simulated tissue 70 and tools 60 that would be visible to the user in an actual surgery). FIG. 5 is an example of the visual representation rendered by another embodiment of the thread 140 on the display 20. In this embodiment, the thread renders not only the tissue 70 and tools 60, but also the remainder of a simulated patient's body 900 and an underlying operating table 910. As shown in FIG. 6, various organs of the patient's body 900 may also be displayed (e.g., various blood vessels, bones, skin, etc. beyond the portion of tissue 70 being directly simulated).

The haptic rendering thread 150 converts the results of the processing threads into information representative of the haptic feedback being applied to the tool(s) 60 as a result of the simulated interaction between the tool(s) 60 and tissue 70 (e.g., resistance to motion of the tool(s) 60 in various translational and rotational directions). The haptic rendering thread 150 provides this information to the haptic output device 90 for conversion into real haptic feedback on the representation(s) 40 of the tool(s) 60.

Distinct visual, physics, and even haptic representations of the same entities in the simulated surgical scene can be interlinked via a multi-resolution world model thread 130 such that much higher spatial resolution visual model can be driven by the behavior of the physics simulation and the haptic feedback provided to the user can also run and a much higher temporal resolution than the physics simulation. In each case, a mapping is preferably done from the physics simulation to the other representations so that a smooth, consistent appearance and feel is perceived by the user as the result of the physics simulation responding to their actions.

As illustrated, the threads are implemented via the program 15 running on the processor 10. However, the program 15 and processor 10 could be integrated into a special-purpose hardware such as a graphics or physics processor. While the threads can be thought of as separate tasks, in practice they may be implemented on a single CPU and be performed serially using the operating system's task scheduler.

The task assignment thread 110 may activate, modify, and/or deactivate threads 115 based on a variety of conditions. For example, various threads 115 may be tool 60 position dependent:
- A physics-enabled tissue anatomy thread 115 may be activated only when a tool 60 is interacting with the simulated tissue 70;
- High resolution threads 115 may be activated with respect to one or more layers 510, 520, 530, 540, 550 of the simulated tissue 70 in the vicinity of the tool 60;
- High resolution "hot spot" threads may track the location of interaction between the tool 60 and the tissue 70 (e.g., maintaining a high resolution thread over the area (for a surface thread) or volume (tissue thread) in the vicinity of the interaction between the tool 60 and the tissue (e.g., a high resolution thread could compute an area or volume within 5 mm, 1 cm, 2 cm, etc. of the interaction between the tool and the model). For example, an FEA thread for one of the layers 510, 520, 530, 540, 550 may change its resolution at a hot spot through tetrahedral splitting to increase the mesh, along with time-based blending of stress and strain to avoid discontinuities in the thread. Similarly, a thread 115 for one of the layers 510, 520, 530, 540, 550 may be split into multiple threads 115 running at different resolutions with the split location being tool 60 position dependent.

The task assignment thread 110 may activate, modify, and/or deactivate threads 115 in response to operation of other threads 115:
- activating a high resolution thread 115 for simulating an end of a ligament any time that a thread 115 operates on any portion of the simulated ligament;
- activating medium resolution threads 115 in response to activation of higher resolution adjacent threads 115 (e.g., providing sequentially lower resolution threads 115 farther and farther away from a location of interaction between the tool 60 and the tissue 70); and/or
- as explained in greater detail below, the task assignment thread may active a blood accumulation thread 115 whenever a blood vessel is cut.

Threads 115 may comprise a variety of computations. Physics threads 115 may calculate how interactions between the tool 60 and the tissue 70 will affect the tissue 70 (e.g., finite element analysis (FEA) threads; biomechanics musculoskeletal mathematical model threads; quantitative systemic physiologic response model threads; blood flow in response to surface wounding or vascular compromise threads) and may compute changes to various aspects of the tissue 70 (e.g., a cloth-type computation to compute movement of the skin surface; a 3D thread to simulate tissue).

Blending threads 115 may be dedicated to blending the results of other threads 115 (e.g., threads with overlapping results; accounting for changes in tissue 70 representation resolution that require consistent time-based response to applied loading) to achieve fast smooth changes to the tissue 70. For example, in one or more embodiments, two overlapping threads 115 cover a portion of the tissue 70 in direct/close proximity to a location of interaction with a tool 60: a first thread 115 has very fast, but physically less accurate computations, while a second thread 115 has slower, more accurate computations of underlying tissue 70 behavior. A blending thread 115 blends the results of these fast, less accurate and slow, more accurate threads 115 to provide a resulting simulation with a more realistic, interactive experience at a small cost in physical accuracy.

Collision detection and response thread(s) 115 may be used to simulate tool 60 interaction with the tissue 70 and suture 60 using several collision detection and response techniques. For example, according to one or more embodiments, hierarchical bounding sphere methods provide reasonable performance relative to accurate contact modeling. Alternatively, direct use of spatially sorted and hierarchically arranged polygonal models can be used. For suture 60 interaction with other tools 60 within PhysX, a combination of polygon-based collision detection and swept-volume detection may be used to ensure that the thin suture 60 does not pass through the narrow tools 60. The response of the various entities (e.g., tools, tissue, suture, etc.) in the simulated surgical scene to the detected collisions can be carried out by a number of approaches that allow physically appropriate behavior to be computed for the interaction forces and/or moments that will be present between the entities. An example is passive force constraints derived from spring-damper elements at the contact location. Another approach is the use of an active constraint calculation such as that done using the linear complementarity problem (LCP) approach where the appropriate contact force/moment is computed that is consistent with the desired contact location of the entities. In addition, these collision response techniques can be used in conjunction with continuous collision detection (CCD) approaches to allow precise calculation of the exact time, location, and force/moment occurring at entity contact so that the time sampled nature of simulated physics does not miss contact between entities. These CCD approaches can lead to iterative substepping of the numerical integrators used in the simulation to make the simulation consistent with the exact moment of contact. Beyond these entity contact situations, PhysBAM and/or additional physics calculations may interact with one another through the modeling of a curved suture needle 60 and its connected thread 60 passing through the tissue 70 relative to the path created by the needle point as it cuts through the tissue. This will lead to constraint forces and/or moments being passed between the needle and/or thread and the simulated tissue such that the tissue deforms as the needle and/or thread is manipulated by tools grasping them and the tools doing the manipulating are subjected to the those same forces and/or moments causing the user to feel their manipulation of the needle and/or thread within the tissue.

As shown in FIG. 4, a bleeding thread 115 calculates blood accumulation and coagulation within and around a position in which a blood vessel such as a vein 700 is pierced/severed. The bleeding thread 115 may calculate a blood-flow rate out of the vein 700 based on a variety of inputs (e.g., the known size, location, standard flow-rate, and/or time variant pressure through the vein 700 at the location of piercing; the extent to which interaction with the tissue 70 causes a constriction in the vein 700, which impedes blood-flow (calculated using well known fluid-flow equations based on the physical properties of blood and blood vessels, e.g., smooth particle hydrodynamics); the size and shape of the hole/cut 710 in the vein 700 (see FIG. 2). The bleeding thread 115 may determine the shape of the pooled blood 720. The calculation may take gravity into consideration, as well as the shape of the surface of the surrounding tissue 70 to determine when blood will pool in a depression such as the incision 730 or flow downhill over the tissue's surface. The bleeding thread 115 may also calculate the time-based coagulation of the blood 720 (e.g., based on time over which the blood 720 is exposed to air), and calculate the blood accumulation/flow accordingly. According to one embodiment, the bleeding thread 115 uses smoothed particle hydrodynamic (SPH)system calculations and Nvidia PhysX to accelerate the calculation of the blood movement. Particle contact and response techniques typical of SPH are used and tuned to compute the movement of blood in free space, such as a strongly bleeding vessel spurting, as well as over surfaces such as sliding over tissue and/or pooling in a depression such as a cut. A separate rendering thread is used to generate an appropriate smooth surface of the blood particle system and to provide coloration over time as the blood coagulates.

Structurally and/or functionally linked threads may be connected to each other such that their results are exchanged. For example, a skin surface thread 115 for the skin layer 510 may be linked to the tissue thread 115 for the tissue layer 520 directly underneath such that changes to either are taken into account in computing changes. There are a variety of ways that this interlinking could be implemented such as force constraints and geometric constraints. Threads 115 may be used to compute the distribution of stress/strain amongst newly structured meshes as the resolution changes in response to tool 60 movement. Threads 115 may be used to distribute the effects of a suture being pulled by a tool 60 that is at a distance from the tissue 70 such that the effects of the suture are properly spread within the tissue 70 through which it passes. Threads 115 can be used to monitor yield criteria of tissue 70 such that if it is overcompressed between tool 60 jaws, pulled too hard or overloaded by suture forces, then the tissue 70 is cut and its topology restructured to reflect the change in its geometry. Threads 115 can be used to monitor tissue 70 loading over time independent of mesh resolution such that the overall loading of the tissue 70 is properly computed and subsequently used in tissue viability estimates.

This physics-based method of simulating the interaction between tool(s) 60 and tissue 70 may provide sufficient information (e.g., stresses, strains, compressive loads, etc.) to determine the impact on the viability of the affected cells within the tissue 70. For example, various cellular viability threads 115 may calculate when interaction with portions of the tissue 70 cause such portions to die.

For example, cellular destruction/viability thread(s) 115 (or one or more of the above-discussed threads 115) may determine whether the localized stresses/strains/compressive loads/tensile loads/etc. within the tissue 70 exceed the mechanical strength, of the internal structure of the cells that would be disposed at particular locations. Such thread(s) 115 may use known cellular properties to calculate such cell morbidity.

As another example, a blood supply thread 115 may associate blood vessels such as the vein 700 with portions of the tissue 70 to which the blood vessel supplies blood. The blood supply thread 115 may itself calculate blood flow (or complete lack thereof) to the associated portions of the tissue 70 (as described above with respect to the bleeding thread 115), or utilize a separate thread for calculating such location-specific blood-flow rate (e.g., the bleeding thread 115). One or more blood supply threads 115 may work in conjunction with one or more bleeding threads 115 to determine the blood flow-rate to various portions of the tissue 70 as a function of the applied force on the tissue and the resulting associated failure of the supply vessels to provide blood. The blood supply thread 115 may also include a clock-function that links the calculated flow rate to the length of time that portions of the tissue 70 receive such flow rate. The blood supply thread 115 may use any suitable model for determining when blood-deprivation causes the death of cells at particular locations within the tissue 70. Such information is generally available in the biomechanics literature as studied tool-tissue interaction. For example, a blood supply thread 115 may calculate when a clamping tool's simulated clamping of a simulated vein 70 deprives downstream cells of oxygen for long enough to kill the cells.

The cellular viability threads 115 may utilize a binary calculation of whether the cells at a particular location within the tissue 70 are alive or dead, or may alternatively determine a range of cell health that spans healthy to dead (e.g., calculating chance of morbidity, an estimate of the percentage of cells within a location that would die, etc.).

The results of the cellular viability threads 115 may be provided in real time to the user through the display 20. For example, the viability threads 115 may cause the display 20 to illustrate areas of reduced cellular viability via localized color changes (e.g., turning white to indicate lack of blood flow; turning blue to indication lack of oxygenated blood; turning darker and darker as viability reduces). Alternatively, the results of the viability threads 115 may be used solely as a means of grading/judging the user's proficiency in the scenario. In such an embodiment, the program 15 may output the results of the viability threads 115 in a qualitatively and/or quantitatively analyzable form (e.g., digital score sheet stored on the processor 10 and/or print out and/or a data log of all state variables that define the surgical scenario at any pointing time during the simulation) for post-simulation critique of the user's actions.

Results of various threads 115 can be transferred to related threads 115 for use by the related thread as they become available (e.g., asynchronously). These results are multidirectional (e.g., results flow between each set of threads whose results may impact the computations of the other thread). This may allow mixing of multiple physics-formulation-based threads 115 that run at different rates, possibly on different cores/CPUs/GPUs of the processor 10. According to one or more embodiments, each thread 115 works at its own rate, publishing results for other threads 115, 140, 150 to use or not depending on the activity within the receiving thread 115 results.

The use of variably operative and variable/varied resolution threads 115 enables computing power to be efficiently utilized where it is most needed to maintain the accuracy of the simulation in real time. For example, the use of a high resolution FEA 3D thread 115 of tissue 70 in the vicinity of a tool 60 helps to provide real time accurate simulation at that critical location, while not wasting such computing power in parts of the model that are less important (e.g., portions of tissue 70 far removed from the tool 60, which therefore undergo little or no tool-based change, and can therefore be accurately simulated with a much lower resolution FEA thread 115).

This approach enables more real time computational speed, which helps to provide a more realistic simulation to a user. The threads 115 operate transparently to the user, who merely provides inputs to the simulator 1 via the representations 40 of the tools 60 and receives visual and/or haptic feedback via the display 20 and/or representations 40 of the tools 60 (via the haptic output devices 90) without knowledge of the complex computational processes being utilized by the simulator 1.

Simulators 1 according to various embodiments of the present invention preferably operate in real time. As used herein, the term "real time" does not require instantaneous computations and user feedback. Rather, "real time" merely means that the simulator provides visual, haptic, and/or other feedback to the user relatively soon after the user provides an input (e.g., within 1 second, within 1/5 of a second, within 1/10 of a second, within 1/15 of a second, within 1/20 of a second, within 1/30 of a second, within 1/60 of a second, within 1/100 of a second, within between 1/5 and 1/100 of a second) and provides it at an update rate that is perceptually relatively smooth. Such computational speed preferably provides a meaningful interactive surgical simulation experience. According to one embodiment, the simulator 1 provides visual feedback within under 100ms of the user providing an input (e.g., manipulating a representation 40 in any direction (e.g., rotation and/or translation in any direction; applying force to a representation 40) and at an update rate of at least 30Hz. Another embodiment would provide haptic feedback response to user interaction with tissue 70 in less than 100ms with an update rate appropriate for the type of material with which the surgical tools 60 are interacting such as 1000Hz for hard surfaces like steel and considerably slower, such as 200Hz, for soft surfaces such as tissue. In general, the lag time and frame rate is preferably consistent with human perceptual capabilities (or relatively close thereto) and preferably does not vary over the course of the simulation to the point where the user can sense something is not as it should be.

For example, it is desirable in various circumstances to run the haptic rendering thread 150 at a faster frequency than a visual rendering thread 140 and underlying simulation because humans sense haptic feedback at a significantly higher frequency than they do visual feedback. According to one embodiment, the haptic rendering thread 150 operates at 1000 Hz while the underlying simulation operates at 30 Hz or less. In this embodiment, the underlying simulation feeds information to the haptic rendering thread 150 at 30 Hz or less and the haptic rendering thread 150 carries a separate localized physics computation that has the effect of smoothing out the information and providing support for stiffer surface interaction via a haptic output at 1000 Hz. The results of the haptic rendering thread 150 are indirectly fed back to the simulation via the user's reactions to the 1000 Hz haptic output, which the simulator 1 receives as inputs to the tool input device 30 (called human-in-the-loop feedback).

As shown in FIG. 3, the simulator 1 enables the simulated grasping by a grasping tool 610 of tissue 70 in a physics-based manner that simulates the real-world interaction with such a grasping tool 610 and tissue 70. The various threads 115 may calculate interaction between the surface of the tool 610 and the tissue 70 to take into effect a variety of factors that affect when, whether, and how grasping the tissue 70 works (e.g., friction between the tool 610 and skin surface of the tissue 70, deformation/bulging of the skin surface as a result of interaction between the tool 610 and the tissue 70, details of the jaw face serrations of the grasping tool 610 affecting the frictional performance between the jaws and the tissue, etc.). These factors combine with a grasping computation thread 115 that can perform the detailed physics calculations local to the tissue 70 near the jaws 610a of the grasping tool 610 to evaluate the jaw-tissue frictional response as well as the detailed tissue response near the jaw 610a faces as the tissue grasping process occurs. This dedication of computational resources paired with the inclusion of sufficiently detailed geometric and behavioral physics produces a simulation of the tissue response to grasping that provides a relatively accurate estimation of real life tool-tissue grasping without resorting to algorithmically predefined behavior that compromises the richness of the simulation in reflecting real world behavior. The grasping physics representation can be defined independent of the tissue surface physics definition if desired or it can be an extension of the physics approach already being used to represent the tissue in the vicinity of the jaws as they contact the tissue. To simplify calculations (though potentially at the expense of some accuracy), the structure of the simulated jaw faces may be simplified, e.g., by reducing or smoothing small surface features such as serrations. However, according to various embodiments, a physics-based grasping feature may be omitted.

To help facilitate the simulation of grasping, a high resolution thread 115 may be activated at each point of contact between the tissue 70 and each jaw 610a (e.g., a local high resolution thread for each location of jaw/tissue interaction). Such high resolution threads 115 may follow the points of contact between the jaws 610a and the tissue 70 when and if they move. As the jaws 610a close, discrete high resolution threads 115 associated with the separate jaws 610a may converge, and be selectively replaced by a single high resolution thread 115.

The dedicated grasping computation threads 115 can model the local deformation of the tissue 70 relative the specific geometric features of the jaw 610a faces as they are pressed into and closed upon the tissue 70 to be grasped. This computation can include details at the appropriate level of detail to allow the mechanical interlock of tissue becoming pushed into the jaw 610a ridges and other geometric shapes as well as frictional contact to result in the tissue 70 being locked into place between the jaws 610a. As this locking of the tissue 70 occurs, additional loading is transferred into the tissue 70 from the jaws 610a and produces increased stress/strain as the tissue 70 is compressed. The models of this local compression phenomena can estimate the level of potential tissue damage from mechanical destruction of cellular structures as well as the indirect damage from cessation of blood cell supporting cellular metabolism. Collectively, this allows local tissue damage resulting from grasping to be evaluated. An additional consequence of this localized interaction modeling is that tissue release modeling can be computed to enable a generalized simulation of the slippage of tissue 70 falling out of the jaws 610a. The combination of detailed physics to allow grasping to be initiated, its effects upon tissue 70 as the grasp is maintained, and the release of tissue 70 provides an appropriately detailed simulation of the physics encountered when a tool is used to hold and manipulate tissue. This same approach can be generalized to produce grabbing of tissue 70 by objects that are pressed into tissue 70 and then slid or moved toward one another. Thus, a grasp and its associated phenomena can be produced even when it is not a single tool whose jaws are closing in on the tissue. Undoubtedly, the single tool and specialized jaw movement and jaw face geometry serve to produce a more effective grasp than would result form two objects pressed together. The important aspect of this being possible in the simulation is that it can be computed can result in appropriate behavior as would occur in real life. Finally, since mechanical structural failure can be estimated relative to cellular failure, this can be expanded to encompass full mechanical failure of the full thickness of tissue 70 within the jaws 610a or between the two objects producing a grasp. Thus, a subsequent rupture and transection of the tissue 70 can be modeled and appropriate changes in tissue 70 topology will result.

As shown in FIG. 3, the simulator 1 enables the simulated cutting of tissue 70 by a scalpel 600. Movement of the scalpel 600 through the tissue severs the tissue 70 along the path of the scalpel 600. The severed path is updated in the various thread(s) 115 that calculate the material being severed (e.g., by severing a constraint connection between portions of a FEA layer on either side of the incision). The threads 115 may also calculate using a yield strength of the tissue 70 relative to the scalpel 600, how much haptic resistance results as the blade overcomes the tissue strength to cut through the tissue 70 (as opposed to preventing movement of the scalpel 600, and or causing the tissue 70 to bunch in front of the scalpel 600 without being cut). The computation of the behavior of the tissue 70 around the scalpel 600 is based on the local contact response of the tissue 70 to the blade faces with the cutting edge 600a capable of generating sufficiently large forces to yield the tissue 70 due to its very small contact area whereas the sides 600b of the blade of the scalpel 600 can only apply much lower forces that will cause the tissue 70 in contact with it to deflect. These blade face contact forces can be distributed over the face such that forces can be generated that resist movement of the blade perpendicular to the blade edge 600a path as well as moments that resist the angulation of the scalpel 600 blade relative to that path as well. These three dimensional forces and moments may provide an appropriate simulation of the user carrying out real world cutting and may be provided as feedback to the user through an appropriate six degree of freedom haptic feedback device. While these detailed computations and haptic feedback may be omitted without deviating from the scope of the present invention, their omission may result in a less realistic experience of the real life cutting behavior. The cutting computation can take place either in the main tissue physics thread 115 or in its own thread 115. The resulting change in the topology of the tissue 70 affected by the cutting is then reflected in the on-going computations of the tissue physics.

Threads 115 to blend results from other threads 115 take into account both differences in the computation rate as well as variations in spatial resolution. Interpolations or extrapolations/projections are used to make sure that the thread 115 observing/receiving the results of another thread 115 gets information that is consistent with its spatial and temporal construction.

The program 15 (and/or a thread within it) may track the user's actions (e.g., the interaction between the tool(s) 60 and tissue 70)) for later analysis and/or grading. The program 15 may also save the state of the surgical scenario so that physics status of the entities active in the interaction of the user with the surgical scenario is known at any moment in time. This information can be used independently or in conjunction with the user actions such that the program 15 may quantitatively compare the user's performance to a known correct interaction for a given scenario to provide feedback/grading to the user. Low-level metrics extracted from the simulator's operation (e.g., the location where a needle or scalpel 60 is inserted into tissue 70, entrance angle of the needle or scalpel 60 relative to the skin or cut surface) can be aggregated to produce refined, objective assessment metrics (e.g., whether the user started the suture or incision in the correct location). The simulator 1 may support automated mentoring capabilities by combining this information in real time with computer generated guidance, which could be in multiple forms either singly or combined such as visual, spoken, haptic, etc., so that the users can effectively progress without having to wait for an experienced instructor to be physically present.

The simulator 1 may support expandable teaching content using rich media (for browser-based presentation), as well as learning management systems that allow remote monitoring of user progress. Surgical scenarios can be created by non-programmers that allow either variations of previous scenarios, e.g. changes in anatomy shape, mechanical response, tool characteristics, suture type, physiological behavior, etc., or development of entirely new scenarios using content creation tools that work similar to widely used computer game content creation tool chains.

While various processes within the simulation are discussed with respect to discrete threads, two or more such threads may be incorporated into a single thread without deviating from the scope of the present invention. For example, a thread 115 used to calculate the shape/deformation of the tissue layer 520 may also calculate when parts of such tissue layer 520 are crushed.

In the illustrated embodiment, the simulator is used to model the interaction between a surgical tool 60 and human tissue 70. However, alternative embodiments of the present invention have far wider application, as the simulator 1 may be used to model a wide variety of objects

The foregoing illustrated embodiments are provided to illustrate the structural and functional principles of the present invention and are not intended to be limiting. To the contrary, the principles of the present invention are intended to encompass any and all changes, alterations and/or substitutions within the spirit and scope of the following claims.

## Claims

1. A method for simulating interaction between a surgical tool and biological tissue, the method comprising:
receiving tool input device information representative of movement of a physical representation of a portion of a simulated surgical tool;
simulating, based on the tool input device information, an interaction between the simulated surgical tool and a simulated biological tissue, the simulating comprising using a plurality of simultaneously running computational threads for calculating the mechanics of the simulated biological tissue, at least some of the threads providing their calculations to one or more associated threads for use in such associated threads' calculations, wherein the plurality of threads comprise at least two threads that have different spatial resolutions, including a first spatial resolution and a second spatial resolution lower than the first resolution, wherein the first higher spatial resolution is utilised in respect of an area where a user is interacting; and
displaying on a display a visual representation of the simulated interaction between the simulated surgical tool and the simulated biological tissue, the visual representation being based on the calculations of the plurality of threads.

2. The method of claim 1, wherein the at least some of the threads provide their calculations to their associated threads asynchronously.

3. The method of claim 1, wherein:
the plurality of threads comprise at least two threads that have different temporal resolutions.

4. The method of claim 1, wherein using the plurality of threads comprises using a moving thread whose spatial coverage on the simulated biological tissue moves in response to simulated movement of the simulated surgical tool, wherein using the moving thread comprises moving the spatial coverage of the moving thread so as to maintain coverage of a moving location of interaction between the simulated surgical tool and the biological tissue.

5. The method of claim 1, wherein:
the plurality of threads comprise at least one selectively activated thread, and
the method further comprises activating and deactivating operation of the at least one selectively activated thread based on a determination of whether the simulated surgical tool's interaction with the simulated biological tissue would impact the calculations of the at least one selectively activated thread by more than a predetermined threshold.

6. The method of claim 1, wherein:
simulating the interaction comprises determining a simulated haptic response based on the calculations of one or more of the threads;
the method further comprises converting the simulated haptic response into a physical haptic response that is applied to the physical representation.

7. The method of claim 6, further comprising providing the physical haptic response to the physical representation at a higher frequency than the simulation and/or visual representation.

8. The method of claim 1, wherein using the plurality of computational threads comprises:
using a first layer thread to calculate changes to a first simulated layer of the biological tissue,
using a second layer thread to calculate changes to a second simulated layer of the biological tissue that adjoins the first simulated layer, and
linking the first and second layer threads to each other so that their adjacent boundaries conform to each other.

9. The method of claim 8, wherein:
the first simulated layer comprises a simulated surface layer, and
the second simulated layer comprises a simulated portion of the biological tissue below the simulated surface layer.

10. The method of claim 1, wherein the represented surgical tool comprises a grasping tool, and wherein simulating comprises simulating a grasping by the tool of a portion of the simulated biological tissue.

11. The method of claim 1, further comprising:
calculating a viability of a portion of the simulated biological tissue based on the simulated interaction, and
visually displaying the calculated viability as part of the visual representation.

12. The method of claim 11, wherein:
the simulating comprises simulating an effect of the simulated interaction on a cellular structure of the simulated biological tissue; and
the calculating of the viability comprises calculating the viability as a function of said effect.

13. The method of claim 11, wherein the calculating of the viability comprises calculating of the viability as a function of blood flow through the simulated biological tissue.

14. A machine-readable medium encoded with machine-executable instructions which, when executed, cause a computer system to simulate interaction between a surgical tool and biological tissue according to a method comprising:
receiving tool input device information representative of movement of a physical representation of a portion of a simulated surgical tool;
simulating, based on the tool input device information, an interaction between the simulated surgical tool and a simulated biological tissue, the simulating comprising using a plurality of simultaneously running computational threads for calculating the mechanics of the simulated biological tissue, at least some of the threads providing their calculations to one or more associated threads for use in such associated threads' calculations, wherein the plurality of threads comprise at least two threads that have different spatial resolutions including a first spatial resolution and a second spatial resolution lower than the first resolution, wherein the first higher spatial resolution is utilised in respect of an area where a user is interacting; and
displaying on a display a visual representation of the simulated interaction between the simulated surgical tool and the simulated biological tissue, the visual representation being based on the calculations of the plurality of threads.

15. A biological tissue simulator comprising:
a physical representation of a portion of a simulated surgical tool;
a tool input device constructed and arranged to measure movement of the physical representation;
a display; and
a computer program operatively connected to the tool input device and the display,
wherein the computer program is programmed to simulate an interaction between the represented surgical tool and a simulated biological tissue, the computer program comprising a plurality of computational threads for calculating the mechanics of the simulated biological tissue, the plurality of computational threads being programmed to run simultaneously, at least some of the threads being programmed to provide their calculations to associated threads for use in such associated threads' calculations, wherein the plurality of threads comprise at least two threads that have different spatial resolutions including a first spatial resolution and a second spatial resolution lower than the first resolution, wherein the first higher spatial resolution is utilised in respect of an area where a user is interacting;
wherein the computer program is programmed to receive from the tool input device information relating to the measured movement and provide the tool input device information to one or more of the threads, and
wherein the computer program is programmed to provide to the display for displaying on the display a visual representation of the simulated interaction between the simulated surgical tool and the simulated biological tissue, the visual representation being based on the calculations of one or more of the threads.

## Patentansprüche

1. Verfahren zum Simulieren einer Interaktion zwischen einem chirurgischen Werkzeug und biologischem Gewebe, das Verfahren umfassend:
Empfangen von Werkzeugeingabevorrichtungsinformationen, die für eine Bewegung einer physischen Darstellung eines Abschnitts eines simulierten chirurgischen Werkzeugs repräsentativ sind;
Simulieren, basierend auf den Werkzeugeingabevorrichtungsinformationen, einer Interaktion zwischen dem simulierten chirurgischen Werkzeug und einem simulierten biologischen Gewebe, wobei das Simulieren das Verwenden einer Vielzahl von gleichzeitig ausgeführten rechnerischen Threads zum Berechnen der Mechanik des simulierten biologischen Gewebes umfasst, wobei mindestens einige der Threads ihre Berechnungen einem oder mehreren zugeordneten Threads zur Verwendung in derartigen Berechnungen zugeordneter Threads bereitstellen, wobei die Vielzahl von Threads mindestens zwei Threads umfasst, die unterschiedliche räumliche Auflösungen aufweisen, die eine erste räumliche Auflösung und eine zweite räumliche Auflösung, die niedriger ist als die erste Auflösung, beinhalten, wobei die erste höhere räumliche Auflösung in Bezug auf einen Bereich verwendet wird, in dem ein Benutzer interagiert; und
Anzeigen einer visuellen Darstellung der simulierten Interaktion zwischen dem simulierten chirurgischen Werkzeug und dem simulierten biologischen Gewebe auf einer Anzeige, wobei die visuelle Darstellung auf den Berechnungen der Vielzahl von Threads basiert.

2. Verfahren nach Anspruch 1, wobei mindestens einige der Threads ihre Berechnungen ihren zugeordneten Threads asynchron bereitstellen.

3. Verfahren nach Anspruch 1, wobei:
die Vielzahl von Threads mindestens zwei Threads umfasst, die unterschiedliche zeitliche Auflösungen aufweisen.

4. Verfahren nach Anspruch 1, wobei das Verwenden der Vielzahl von Threads das Verwenden eines sich bewegenden Threads umfasst, dessen räumliche Abdeckung auf dem simulierten biologischen Gewebe sich in Reaktion auf eine simulierte Bewegung des simulierten chirurgischen Werkzeugs bewegt, wobei das Verwenden des sich bewegenden Threads das Bewegen der räumlichen Abdeckung des sich bewegenden Threads umfasst, um eine Abdeckung einer sich bewegenden Position der Interaktion zwischen dem simulierten chirurgischen Werkzeug und dem biologischen Gewebe beizubehalten.

5. Verfahren nach Anspruch 1, wobei:
die Vielzahl von Threads mindestens einen selektiv aktivierten Thread umfasst und
das Verfahren ferner das Aktivieren und Deaktivieren einer Operation des mindestens einen selektiv aktivierten Threads basierend auf einer Ermittlung umfasst, ob die Interaktion des simulierten chirurgischen Werkzeugs mit dem simulierten biologischen Gewebe die Berechnungen des mindestens einen selektiv aktivierten Threads um mehr als einen vorbestimmten Schwellenwert beeinflussen würde.

6. Verfahren nach Anspruch 1, wobei:
das Simulieren der Interaktion das Ermitteln einer simulierten haptischen Reaktion basierend auf den Berechnungen von einem oder mehreren der Threads umfasst;
das Verfahren ferner das Umwandeln der simulierten haptischen Reaktion in eine physische haptische Reaktion umfasst, die auf die physische Darstellung angewendet wird.

7. Verfahren nach Anspruch 6, ferner umfassend das Bereitstellen der physischen haptischen Reaktion auf die physische Darstellung bei einer höheren Frequenz als die Simulation und/oder visuelle Darstellung.

8. Verfahren nach Anspruch 1, wobei das Verwenden der Vielzahl von rechnerischen Threads umfasst:
Verwenden eines ersten Schichten-Threads, um Veränderungen an einer ersten simulierten Schicht des biologischen Gewebes zu berechnen,
Verwenden eines zweiten Schichten-Threads, um Veränderungen an einer zweiten simulierten Schicht des biologischen Gewebes, das an die erste simulierte Schicht angrenzt, zu berechnen und
Verbinden der Threads der ersten und der zweiten Schicht miteinander, sodass ihre benachbarten Grenzen übereinstimmen.

9. Verfahren nach Anspruch 8, wobei:
die erste simulierte Schicht eine simulierte Oberflächenschicht umfasst und
die zweite simulierte Schicht einen simulierten Abschnitt des biologischen Gewebes unterhalb der simulierten Oberflächenschicht umfasst.

10. Verfahren nach Anspruch 1, wobei das dargestellte chirurgische Werkzeug ein Greifwerkzeug umfasst und wobei das Simulieren das Simulieren eines Ergreifens eines Abschnitts des simulierten biologischen Gewebes durch das Werkzeug umfasst.

11. Verfahren nach Anspruch 1, ferner umfassend:
Berechnen einer Funktionsfähigkeit eines Abschnitts des simulierten biologischen Gewebes basierend auf der simulierten Interaktion und
visuelles Anzeigen der berechneten Funktionsfähigkeit als Teil der visuellen Darstellung.

12. Verfahren nach Anspruch 11, wobei:
das Simulieren das Simulieren einer Wirkung der simulierten Interaktion auf einer Zellenstruktur des simulierten biologischen Gewebes umfasst; und
das Berechnen der Funktionsfähigkeit das Berechnen der Funktionsfähigkeit in Abhängigkeit von der Wirkung umfasst.

13. Verfahren nach Anspruch 11, wobei das Berechnen der Funktionsfähigkeit das Berechnen der Funktionsfähigkeit in Abhängigkeit von dem Blutstrom durch das simulierte biologische Gewebe umfasst.

14. Maschinenlesbares Medium, das mit maschinenausführbaren Anweisungen codiert ist, die bei Ausführung bewirken, dass ein Computersystem eine Interaktion zwischen einem chirurgischen Werkzeug und biologischem Gewebe gemäß einem Verfahren simuliert, das umfasst:
Empfangen von Werkzeugeingabevorrichtungsinformationen, die für eine Bewegung einer physischen Darstellung eines Abschnitts eines simulierten chirurgischen Werkzeugs repräsentativ sind;
Simulieren einer Interaktion zwischen dem simulierten chirurgischen Werkzeug und einem simulierten biologischen Gewebe, basierend auf den Werkzeugeingabevorrichtungsinformationen, wobei das Simulieren das Verwenden einer Vielzahl von gleichzeitig ausgeführten rechnerischen Threads zum Berechnen der Mechanik des simulierten biologischen Gewebes umfasst, wobei mindestens einige der Threads ihre Berechnungen einem oder mehreren zugeordneten Threads zur Verwendung in derartigen Berechnungen von Threads bereitstellen, wobei die Vielzahl von Threads mindestens zwei Threads umfasst, die unterschiedliche räumliche Auflösungen aufweisen, die eine erste räumliche Auflösung und eine zweite räumliche Auflösung beinhalten, die niedriger ist als die erste Auflösung, wobei die erste höhere räumliche Auflösung in Bezug auf einen Bereich verwendet wird, in dem ein Benutzer interagiert; und
Anzeigen einer visuellen Darstellung der simulierten Interaktion zwischen dem simulierten chirurgischen Werkzeug und dem simulierten biologischen Gewebe auf einer Anzeige, wobei die visuelle Darstellung auf den Berechnungen der Vielzahl von Threads basiert.

15. Simulator von biologischem Gewebe, umfassend:
eine physische Darstellung eines Abschnitts eines simulierten chirurgischen Werkzeugs;
eine Werkzeugeingabevorrichtung, die konstruiert und angeordnet ist, um eine Bewegung der physischen Darstellung zu messen;
eine Anzeige; und
ein Computerprogramm, das funktionsmäßig mit der Werkzeugeingabevorrichtung und der Anzeige verbunden ist,
wobei das Computerprogramm programmiert ist, um eine Interaktion zwischen dem dargestellten chirurgischen Werkzeug und einem simulierten biologischen Gewebe zu simulieren, das Computerprogramm eine Vielzahl von rechnerischen Threads zum Berechnen der Mechanik des simulierten biologischen Gewebes umfasst, die Vielzahl von rechnerischen Threads programmiert ist, um gleichzeitig ausgeführt zu werden, wobei mindestens einige der Threads programmiert sind, um ihre Berechnungen zugeordneten Threads zur Verwendung in derartigen Berechnungen zugeordneter Threads bereitzustellen, wobei die Vielzahl von Threads mindestens zwei Threads umfasst, die unterschiedliche räumliche Auflösungen aufweisen, die eine erste räumliche Auflösung und eine zweite räumliche Auflösung beinhalten, die niedriger ist als die erste Auflösung, wobei die erste höhere räumliche Auflösung in Bezug auf einen Bereich verwendet wird, in dem ein Benutzer interagiert;
wobei das Computerprogramm programmiert ist, um von der Werkzeugeingabevorrichtung Informationen betreffend die gemessene Bewegung zu empfangen und der Werkzeugeingabevorrichtung Informationen einem oder mehreren der Threads bereitzustellen, und
wobei das Computerprogramm programmiert ist, um der Anzeige zum Anzeigen auf der Anzeige eine visuelle Darstellung der simulierten Interaktion zwischen dem simulierten chirurgischen Werkzeug und dem simulierten biologischen Gewebe auf der Anzeige bereitzustellen, wobei die visuelle Darstellung auf den Berechnungen eines oder mehrerer der Threads basiert.

## Revendications

1. Procédé de simulation d'interaction entre un outil chirurgical et un tissu biologique, le procédé comprenant :
recevoir des informations de dispositif d'entrée d'outil représentant un mouvement d'une représentation physique d'une partie d'un outil chirurgical simulé ;
simuler, sur la base des informations de dispositif d'entrée d'outil, une interaction entre l'outil chirurgical simulé et un tissu biologique simulé, la simulation comprenant utiliser une pluralité de fils de calcul en exécution simultanée pour calculer la mécanique du tissu biologique simulé, au moins certains des fils fournissant leurs calculs à un ou plusieurs fils associés pour une utilisation dans des calculs de ces fils associés, la pluralité de fils comprenant au moins deux fils qui ont des résolutions spatiales différentes, comprenant une première résolution spatiale et une seconde résolution spatiale inférieure à la première résolution, la première résolution spatiale plus élevée étant utilisée pour une zone dans laquelle un utilisateur interagit ; et
afficher, sur un dispositif d'affichage, une représentation visuelle de l'interaction simulée entre l'outil chirurgical simulé et le tissu biologique simulé, la représentation visuelle étant basée sur les calculs de la pluralité de fils.

2. Procédé selon la revendication 1, dans lequel au moins certains des fils fournissent leurs calculs à leurs fils associés de manière asynchrone.

3. Procédé selon la revendication 1, dans lequel :
la pluralité de fils comprennent au moins deux fils qui ont des résolutions temporelles différentes.

4. Procédé selon la revendication 1, dans lequel l'utilisation de la pluralité de fils comprend utiliser un fil mobile dont la couverture spatiale sur le tissu biologique simulé se déplace en réponse à un mouvement simulé de l'outil chirurgical simulé, l'utilisation du fil mobile comprenant déplacer la couverture spatiale du fil mobile de façon à maintenir la couverture d'un emplacement d'interaction mobile entre l'outil chirurgical simulé et le tissu biologique.

5. Procédé selon la revendication 1, dans lequel :
la pluralité de fils comprennent au moins un fil activé de manière sélective, et
le procédé comprend en outre activer et désactiver le fonctionnement de l'au moins un fil activé de manière sélective, par détermination du point de savoir si l'interaction de l'outil chirurgical simulé avec le tissu biologique simulé affecterait les calculs de l'au moins un fil activé de manière sélective au-delà d'un seuil prédéterminé.

6. Procédé selon la revendication 1, dans lequel :
la simulation de l'interaction comprend déterminer une réponse haptique simulée sur la base des calculs d'un ou plusieurs des fils ;
le procédé comprend en outre convertir la réponse haptique simulée en une réponse haptique physique qui est appliquée à la représentation physique.

7. Procédé selon la revendication 6, comprenant en outre fournir la réponse haptique physique à la représentation physique à une fréquence plus élevée que la simulation et/ou la représentation visuelle.

8. Procédé selon la revendication 1, dans lequel l'utilisation de la pluralité de fils de calcul comprend :
utiliser un fil de première couche pour calculer des changements d'une première couche simulée du tissu biologique,
utiliser un fil de seconde couche pour calculer des changements d'une seconde couche simulée du tissu biologique, qui est adjacente à la première couche simulée, et
lier les fils de première et seconde couches l'un à l'autre de telle sorte que leurs limites adjacentes correspondent l'une à l'autre.

9. Procédé selon la revendication 8, dans lequel :
la première couche simulée comprend une couche de surface simulée, et
la seconde couche simulée comprend une partie simulée du tissu biologique au-dessous de la couche de surface simulée.

10. Procédé selon la revendication 1, dans lequel l'outil chirurgical représenté comprend un outil de préhension, et la simulation comprenant simuler une préhension, par l'outil, d'une partie du tissu biologique simulé.

11. Procédé selon la revendication 1, comprenant en outre :
calculer une viabilité d'une partie du tissu biologique simulé sur la base de l'interaction simulée, et
afficher visuellement la viabilité calculée en tant que partie de la représentation visuelle.

12. Procédé selon la revendication 11, dans lequel :
la simulation comprend simuler un effet de l'interaction simulée sur une structure cellulaire du tissu biologique simulé ; et
le calcul de la viabilité comprend calculer la viabilité en fonction dudit effet.

13. Procédé selon la revendication 11, dans lequel le calcul de la viabilité comprend calculer la viabilité en fonction du débit sanguin à travers le tissu biologique simulé.

14. Support lisible par machine, codé avec des instructions exécutables par machine qui, lorsqu'elles sont exécutées, amènent un système informatique à simuler une interaction entre un outil chirurgical et un tissu biologique selon un procédé comprenant :
recevoir des informations de dispositif d'entrée d'outil représentant un mouvement d'une représentation physique d'une partie d'un outil chirurgical simulé ;
simuler, sur la base des informations de dispositif d'entrée d'outil, une interaction entre l'outil chirurgical simulé et un tissu biologique simulé, la simulation comprenant utiliser une pluralité de fils de calcul en exécution simultanée pour calculer la mécanique du tissu biologique simulé, au moins certains des fils fournissant leurs calculs à un ou plusieurs fils associés pour une utilisation dans des calculs de ces fils associés, la pluralité de fils comprenant au moins deux fils qui ont des résolutions spatiales différentes, comprenant une première résolution spatiale et une seconde résolution spatiale inférieure à la première résolution, la première résolution spatiale plus élevée étant utilisée pour une zone dans laquelle un utilisateur interagit ; et
afficher, sur un dispositif d'affichage, une représentation visuelle de l'interaction simulée entre l'outil chirurgical simulé et le tissu biologique simulé, la représentation visuelle étant basée sur les calculs de la pluralité de fils.

15. Simulateur de tissu biologique comprenant :
une représentation physique d'une partie d'un outil chirurgical simulé ;
un dispositif d'entrée d'outil configuré et agencé pour mesurer un mouvement de la représentation physique ;
un dispositif d'affichage ; et
un programme d'ordinateur connecté de manière fonctionnelle au dispositif d'entrée d'outil et au dispositif d'affichage,
le programme d'ordinateur étant programmé pour simuler une interaction entre l'outil chirurgical représenté et un tissu biologique simulé, le programme d'ordinateur comprenant une pluralité de fils de calcul pour calculer la mécanique du tissu biologique simulé, la pluralité de fils de calcul étant programmés pour s'exécuter simultanément, au moins certains des fils étant programmés pour fournir leurs calculs à des fils associés pour une utilisation dans des calculs de ces fils associés, la pluralité de fils comprenant au moins deux fils qui ont des résolutions spatiales différentes, comprenant une première résolution spatiale et une seconde résolution spatiale inférieure à la première résolution, la première résolution spatiale plus élevée étant utilisée pour une zone dans laquelle un utilisateur interagit ;
le programme d'ordinateur étant programmé pour recevoir, en provenance du dispositif d'entrée d'outil, des informations relatives au mouvement mesuré et fournir les informations de dispositif d'entrée d'outil à un ou plusieurs des fils, et
le programme d'ordinateur étant programmé pour fournir, au dispositif d'affichage, pour afficher sur le dispositif d'affichage, une représentation visuelle de l'interaction simulée entre l'outil chirurgical simulé et le tissu biologique simulé, la représentation visuelle étant basée sur les calculs d'un ou plusieurs des fils.
